# EUROPEAN PATENT SPECIFICATION

(11) **EP 4 353 197 B1**
(45) Date of publication and mention of the grant of the patent: **31.12.2025**
(21) Application number: 22201675.0
(22) Date of filing: 14.10.2022
(51) Int. Cl.: A61F 2/44

(54) **INTERVERTEBRAL DISK PROSTHESIS**
BANDSCHEIBENPROTHESE
PROTHESE DE DISQUE INTERVERTEBRAL

(43) Date of publication of application: 17.04.2024
(73) Proprietor: Marnay, Thierry, 34090 Montpellier (FR); Cloix, Erick, 33970 Cap Ferret (FR)
(72) Inventor: Marnay, Thierry, 34090 Montpellier (FR); Cloix, Erick, 33970 Cap Ferret (FR)
(74) Representative: Yes My Patent

(56) References cited:
- EP-A1- 0 277 282
- WO-A1-2008/131498
- WO-A2-02/17825
- WO-A2-2006/060482
- KR-A- 20130 068 860
- US-A1- 2010 100 185
- US-A1- 2010 234 954

## Description

The present invention relates to the field of intervertebral prosthesis. In particular to the field of devices which can be used when intervertebral disks are damaged.

Intervertebral disks, sometimes also referred to as intervertebral fibrocartilage, are structures comprised between adjacent vertebrae which can be deformed when one vertebra moves relatively to another vertebra, thus acting both as a joint and as a shock absorber.

Indeed, depending on the movement intervertebral disks can be submitted to very high pressures, especially in case of heavy people, as a significant part of the weight can eventually be absorbed by the intervertebral disks.

In order to face such dramatic constraints, intervertebral disks present a central gel-like nucleus and a fibrous outer ring. The nucleus can redistribute the pressure applied to the disk in all directions whereas the fibrous ring can withstand high compressive forces.

When the intervertebral disk is dysfunctional, e.g. in case of a herniation or complete degeneration, which can be caused by intense and repeated stress of the disk, or by a height loss due to dehydration of the nucleus, a need for prosthesis may arise so as to avoid surgically fusing the vertebrae.

However, due to the high pressures which are to be withstood, mimicking natural structures is not an option. Indeed, without natural regeneration, such structures would not be able to last long enough.

Prosthesis also need to be of low encumbrance. Indeed, due to their size existing prosthesis often need to be set up through the belly, thus requiring two surgeons, one for the guts and one for the spine, making it the surgery very expensive. This is mostly true for the lumbar prosthesis as cervical vertebrae are easier to access.

Existing prosthesis usually make contact with the vertebrae through a titanium convex shape treated to hang properly with the bones. On the long run however, metallic
structures tend to form cations which are cancerogenic, making those structures unsuitable. US 2010/234954 A1 and KR 2013 0068860 A disclose examples of Intervertebral disk prosthesis with multiple interconnected and fluid filled compartments distributed in an deformable casing.

Therefore, known prosthesis are usually made of a deformable chamber filled with a liquid or a gel which can absorb the pressure applied in case of a deformation. Such prosthesis present several issues. Most importantly, the kinetics to absorb the deformation and to return to its initial shape when the pressure is suppressed is not handled in a satisfying manner. Furthermore, pressure tends to be applied heterogeneously on the disk, and regular prosthesis allow only for an homogeneous response, which can be inadequate and, eventually, damage the vertebrae. Lastly, such prosthesis cannot withstand well very high pressure, such as the one caused by heavy people squatting down.

There is thus a need for an intervertebral prosthesis able to provide an heterogeneous response to heterogeneous pressure, which can withstand high pressure, and presents a deformation kinetics adapted to physiological conditions.

The present invention provides an answer to these needs and relates to an intervertebral disk prosthesis comprising an external waterproof multilayered deformable casing surrounding a peripheral partition membrane delimiting an outer space and an inner cavity,
wherein the inner cavity is partitioned by vertical walls delimiting at least four cavity compartments configured to be filled with a non-toxic saline solution, and
wherein the vertical walls comprise orifices and/or porous membranes configured to induce the saline solution to flow from one cavity compartment toward another upon heterogeneous application of a pressure comprised between 0.05 and 3 MPa in a duration comprised between 10 seconds and 60 minutes, and to revert back to its native compartment upon removal of said pressure in a duration comprised between 10 seconds and 180 minutes, and
wherein the walls are distributed asymmetrically in the inner cavity so that a posterior part of the inner cavity comprises fewer and/or larger cavity compartments than an anterior part of the inner cavity.

Waterproof is to be understood as able to contain the fluids which are to be in contact with the casing in the conditions in which the prosthesis is to be used, especially high pressure. Indeed, it is of utmost importance to prevent leakage which would ruin the properties of the device.

Deformable is to be understood as able to take a different shape when pressure is applied on it.

It is the overall multilayered casing which is to be deformable and waterproof. However, it can comprise at least one non-waterproof layer, or partial layer, which allow fluid circulation inside the casing. Similarly, it can comprise non deformable parts which can constitute a backbone of the casing and orient its overall deformation. Preferably, it comprises an internal flexible layer which provides the waterproofness and an external, thicker layer which gives the casing its shape and overall mechanical properties.

The partition membrane defines the inner cavity and bears the vertical walls which define compartments. The partition membrane has a lower part and an upper part. Vertical is to be understood as linking these two parts or arising from any of these two parts, but a wall does not need to be parallel to the vertical axis to be considered a vertical wall. It can indeed present an angle with respect to the vertical axis, which can be variable depending on the portion of the wall.

The overall device typically presents a depth, that is to say a dimension along the antero-posterior axis of the bearer, comprised between 20 and 36 mm, a width comprised between 24 and 40 mm, and a height comprised between 8 and 14 mm, preferably between 8 and 12 mm.

These dimensions need not be constant on the whole prosthesis. Although the device is called an intervertebral disc prosthesis, it does not have the shape of an exact disc. The term disc is used in reference to the anatomic structure the prosthesis is meant to replace. For instance, the device is preferably provided with low height zones and higher height zones which allow to control the overall fluid circulation and pressure response.

A compartment is defined by a zone in which the fluid contained in the inner cavity can circulate without mechanical impairment. When the walls do not present any orifice, or when the orifice/pores are small enough, it is easy to define the compartments. However, when the orifices grow larger, especially when their size takes all the height of the cavity, thus defining a complete interruption in the vertical wall, compartments definition can become trickier. It is also true in case of vertical walls which do not link the upper and lower parts together, which can also be referred to as "half-walls", or in case of vertical walls which end mid-way without completely closing a chamber.

In such litigious cases, two zones between which the fluid contained in the inner cavity can circulate without going through any orifice or pore are to be considered as belonging to the same compartment. An orifice is a recess in a vertical wall which smallest dimension is inferior to the height of the inner cavity at its position. For instance, a hole in a vertical wall is an orifice. Similarly, the previously called half-walls which arise from the upper or lower part of the inner cavity without linking it to its counterpart do define an orifice corresponding to the part between the end of the wall and said counterpart, which smallest dimension is indeed inferior to the height of the cavity.

In a more limited embodiment of the invention, the orifices present a highest dimension comprised between 1 and 6 mm. Preferably, all the orifices do not have the same size. Orifices can have a round or angular shape, e.g. selected from an oval shape, a trapezoidal shape, or a polygonal shape such as star shape.

A porous or microporous membrane can also be used with smaller pores with a greater density so as to present similar fluid circulation through the wall in a more homogeneous way.

The non toxic saline solution must be a solution which would not be harmful in case of accidental leakage of the prosthesis. Preferably, the non toxic saline solution is physiological serum. The solution can be completely liquid or can be dispersed into a hydrogel. It can also comprise additive, either for conservation or to tune its mechanical properties. For instance, it can comprise compounds which increase its viscosity.

Heterogeneous application of pressure is to be understood as an amount of applied pressure which is not the same on all the parts of the prosthesis.

Values of 0.05 and 3 MPa applied during between 10 seconds and 60 minutes correspond to standard pressure to which a disc is exposed in physiological conditions.

Reverting back to its native compartment upon removal of said pressure in a duration comprised between 10 seconds and 180 minutes correspond to a suitable kinetic allowing to mimic a regular intervertebral disc with the support of the remaining healthy vertebral column.

Preferably the external waterproof deformable casing comprises four layers. In order to maintain the shape, at least one of the layers can be structured in two different directions. Preferably, the most external layer is a thick and solid layer, the most internal layer is flexible and waterproof, and the two intermediate layers are used as a radial backbone.

Preferably, the walls are anchored into the most external layer of the external waterproof deformable casing, which allows to stabilize the walls and to prevent them to drift upon deformation.

Preferably, the device comprises a number of walls in the antero-posterior plane comprised between 1 and 8.

Preferably, the device comprises a number of walls in frontal plane comprised between 1 and 8.

In an advantageous embodiment, the inner cavity comprises pressure sensors. The sensors can allow monitoring the pressure and detect defect in the prosthesis. Preferably, the sensors can communicate with an external device wirelessly.

The most external layer of the external waterproof deformable casing is preferably reinforced with a rigid material, preferably selected from polyester and polyethylene, used separately or in combination. Such materials allow for optimal and tunable mechanical properties whilst presenting good biocompatibility.

Preferably, the vertical walls comprise at least one vertical wall presenting a multiply folded shape conferring spring-like properties. Such properties allow a higher response to pressure deformation and quicker return to its native shape upon deformation.

Preferably, at least one vertical wall presents a tubular shape, which can trap part of the liquid so as to restrain its circulation and further tune the mechanical properties of the prosthesis upon deformation.

Preferably, the water density in the inner cavity is constant.

Preferably the vertical walls comprise orifices with a larger dimension comprised between 1 mm and 6 mm.

Preferably the external waterproof deformable casing is made of a 3D printable material.

Preferably the vertical walls are made of the same material than the peripheral partition membrane. The vertical walls and the peripheral partition membrane can even be molded or made of a single piece, to provide optimal waterproofness.

Preferably, the intervertebral disk prosthesis comprises sensors able to measure parameters of the intervertebral disk prothesis and wirelessly communicate the measured value to an external device, wherein said parameters are preferably selected from pressure, pH, and salinity. Such measurements can allow detection of leakage or damages inside the prosthesis so as to decide proactively whether there is a need to replace the prosthesis or not.

Another object of the present invention is a disposable syringe for filling an intervertebral disk prothesis according to the present invention, comprising an external chamber configured to contain the non-toxic saline solution, and an internal chamber configured to receive a pressure sensor able to measure the pressure inside the inner cavity during its filling. Indeed, the prosthesis can be positioned empty or full, depending on the location. When the prosthesis needs to be filled *in situ,* it is of utmost importance to have very accurate control of the process, in particular of the pressure inside the inner cavity so as to avoid overfilling or underfilling of the cavity.

A probe is preferably used to monitor the pressure during the positioning of the prothesis according to the invention.

The probe is preferably left in place at the end of the positioning as its retreat can be hazardous and as it could prove useful in case a further intervention is needed.

The invention also relates to a medical treatment consisting in replacing a defective intervertebral disk of a patient with a intervertebral disk prothesis according to the present invention, wherein the maximum pressure applied on the disk without lifting any charge is calculated based on the weight and height of the patient and the dimensions of the orifices are configured to induce the saline solution to flow from one cavity compartment toward another upon heterogeneous application of said calculated pressure in a duration comprised between 10 seconds and 60 minutes, and to revert back to its native compartment upon removal of said pressure in a duration comprised between 10 seconds and 180 minutes.

The present invention shall be better understood based on the following detailed description of a non-limiting embodiment of the present invention, and on the annexed drawing on which:
- figure 1 is a across sectional view of a device according to the invention, and 25
- figure 2 is a perspective view of a device according to the invention without the upper part of the partition membrane. For clarity sake, the relative sizes and proportions of the elements depicted on the drawing have not always been respected, it is understood that the depicted views are merely schematic.

In an exemplary embodiment, it is provided a prosthesis with an injectable balloon presenting an external waterproof membrane 1. The membrane 1 is enclosed in a non-illustrated four layered casing.

Said casing is made of woven polyester which reproduces the annular circulation of the disc and is coated with a biological adhesive layer which allows proper adhesion to the articular tissues.

The waterproof membrane 1 defines an internal cavity filled with physiological serum. The waterproof membrane presents a lower part 4 and an upper part which is not illustrated.

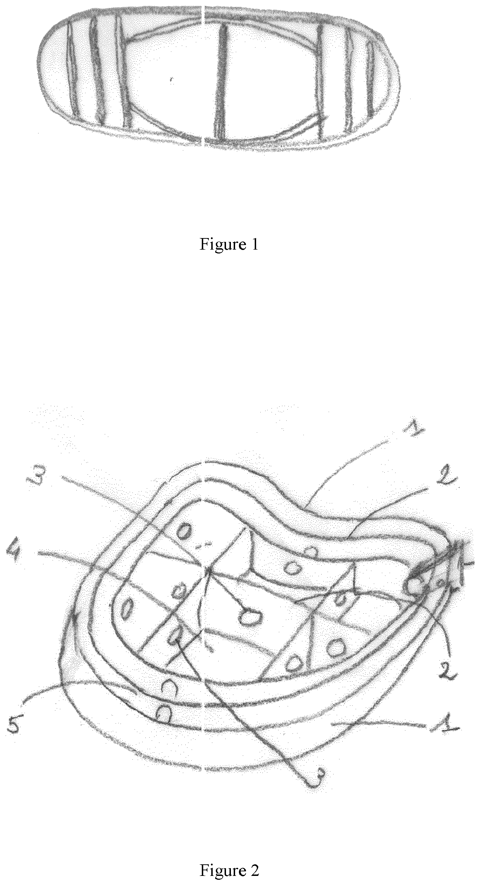

As opposed to existing prosthesis which are filled with silicone, physiological serum is harmless in case of leakage. In order to get a proper rheology, circulation of the physiological serum is hampered and organized by internal walls 2. Some of these walls are vertical while others are of horizontal orientation as depicted on figures 1 and 2, thus defining a plurality of compartments, such as compartment 5.

Walls present oval apertures 3 which allow fluid circulation between the compartments.

The casing and the partition membrane are 3D printed based on imaging data of the patient, allowing for a personalized prosthesis. The casing and the membrane are thus made of materials which are both biocompatible and 3D printable, such as polyethylene, silicone or polyurethane.

The pressure inside the prosthesis must not exceed the pressure of a disc, i.e. comprised between 200 and 300 kPa, which is made possible by the fact that the fluid used is physiological serum, as opposed to silicone in prior art. The pressure inside the disc is thus controlled during its filling by appropriate material. Advantageously, pressure sensors able to communicate wirelessly with an external device are used to monitor the pressure at all times.

The prosthesis is positioned empty through a cannula and then filled in situ through a valve, which allows to avoid belly surgery.

For cervical prosthesis however, it is possible to position a prefilled prosthesis as there is no such constraints.

The filling speed, the volume and the target pressure are preestablished based on the size of the prosthesis and of the weight of the patient. The shape of the filled disc is designed to be a substitute to the normal disc but ideally also spreads on the vertebrae in a bean shape with rounded vertical faces.

Walls 2 are oriented so as to maintain the disc in place. Indeed, the pressure applied to the envelope could cause a rolling effect which must be avoided. The disc must also be able to resist the shearing stress applied laterally, rotationally, and along the anteroposterior axis.

The prosthesis according to the present embodiment deforms upon compression and returns to its original shape when the pressure is released. The return operates between 5 and 10 minutes.

In order to mimic the mechanics of a natural intervertebral disc, the walls and the apertures are configured to ease the fluid circulation toward the front: there are less walls and more opening in the front part of the prosthesis to favor a laterally triangular compression.

The walls 2 and the openings 3 are positioned depending on the biomechanical dynamics of the site where the prosthesis is to be positioned, and depending on the weight of the patient. The walls can be continuously linked to the partition membrane 1 or can present loose parts.

The fibers of the walls 2 can be either vertical, horizontal or oblique and their density, orientation and structure can either be constant or vary depending on their application potential.

The internal walls are configured to allow the full circulation of the fluid and do not act as dialysis membrane.

The positioning of the prosthesis according to the present embodiment is done to replace an intervertebral disc after exeresis of disc tissue with instrumentation for height adjustment, trial template using an inflatable balloon of the same shape, and centering and positioning control using radiopaque markers.

In an alternative embodiment, the prosthesis according to the present invention can be inserted intra-articularly in intervertebral discs, posterior vertebral joints such as facet joints or zygapophyses; while this type of application is not claimed, the prosthesis may be suitable to be inserted in elbow, wrist, hip, knee, ankle, talus and any other joint location of the locomotor system.

The invention is not limited to the described embodiments. Unless stated otherwise, the expression "comprising one" is to be understood as "comprising at least one" and "or" is to be understood as "and/or.

## Claims

1. Intervertebral disk prosthesis comprising an external waterproof multilayered deformable casing surrounding a peripheral partition membrane (1) delimiting an outer space and an inner cavity,
wherein the inner cavity is partitioned by vertical walls (2) delimiting at least four cavity compartments (5) configured to be filled with a non-toxic saline solution, and
wherein the vertical walls (2) comprise orifices (3) and/or porous membranes configured to induce the saline solution to flow from one cavity compartment (5) toward another upon heterogeneous application of a pressure comprised between 0.05 and 3 MPa in a duration comprised between 10 seconds and 60 minutes, and to revert back to its native compartment (5) upon removal of said pressure in a duration comprised between 10 seconds and 180 minutes, and
wherein the walls (2) are distributed asymmetrically in the inner cavity so that a posterior part of the inner cavity comprises fewer and/or larger cavity compartments (5) than an anterior part of the inner cavity;
wherein the walls (2) are anchored into the most external layer of the external waterproof deformable casing.

2. Intervertebral disk prothesis according to claim 1, **characterized in that** the external waterproof deformable casing comprises four layers.

3. Intervertebral disk prothesis according to any of the preceding claims, **characterized in that** the inner cavity comprises pressure sensors.

4. Intervertebral disk prothesis according to any of the preceding claims, **characterized in that** the most external layer of the external waterproof deformable casing is reinforced with a rigid material, preferably selected from polyester and polyethylene, used separately or in combination.

5. Intervertebral disk prothesis according to any of the preceding claims, **characterized in that** the vertical walls (2) comprise at least one vertical wall presenting a multiply folded shape conferring spring-like properties.

6. Intervertebral disk prothesis according to any of the preceding claims, **characterized in that** the vertical walls (2) comprise at least one vertical wall presenting a tubular shape.

7. Intervertebral disk prothesis according to any of the preceding claims, **characterized in that** the water density in the inner cavity is constant.

8. Intervertebral disk prothesis according to any of the preceding claims, **characterized in that** the vertical walls (2) comprise orifices (3) with a larger dimension comprised between 1 mm and 6 mm.

9. Intervertebral disk prothesis according to any of the preceding claims, **characterized in that** the external waterproof deformable casing is made of a 3D printable material.

10. Intervertebral disk prothesis according to any of the preceding claims, **characterized in that** the vertical walls (2) are made of the same material than the peripheral partition membrane (1).

11. Intervertebral disk prothesis according to any of the preceding claims, **characterized in that** the non-toxic saline solution is physiological serum.

12. Intervertebral disk prothesis according to any of the preceding claims, **characterized in that** it comprises sensors able to measure parameters of the intervertebral disk prothesis and wirelessly communicate the measured value to an external device, wherein said parameters are preferably selected from pressure, pH, and salinity.

## Patentansprüche

1. Bandscheibenprothese, umfassend eine äußere wasserdichte mehrschichtige verformbare Hülle, die eine periphere Trennmembran (1) umgibt, die einen äußeren Raum und einen inneren Hohlraum abgrenzt,
wobei der innere Hohlraum durch vertikale Wände (2) getrennt ist, die mindestens vier Hohlraumfächer (5) abgrenzen, die konfiguriert sind, um mit einer ungiftigen Salzlösung gefüllt zu werden, und
wobei die vertikalen Wände (2) Öffnungen (3) und/oder poröse Membranen umfassen, die konfiguriert sind, um die Salzlösung zu veranlassen, bei heterogener Anwendung eines Drucks zwischen 0,05 und 3 MPa für eine Dauer zwischen 10 Sekunden und 60 Minuten von einem Hohlraumfach (5) zu einem anderen zu fließen und um bei Entfernung des Drucks für eine Dauer zwischen 10 Sekunden und 180 Minuten in ihr ursprüngliches Fach (5) zurückzukehren, und
wobei die Wände (2) in dem inneren Hohlraum asymmetrisch verteilt sind, sodass ein hinterer Teil des inneren Hohlraums weniger und/oder größere Hohlraumfächer (5) umfasst als ein vorderer Teil des inneren Hohlraums;
wobei die Wände (2) in der äußersten Schicht der äußeren wasserdichten verformbaren Hülle verankert sind.

2. Bandscheibenprothese nach Anspruch 1, **dadurch gekennzeichnet, dass** die äußere wasserdichte verformbare Hülle vier Schichten umfasst.

3. Bandscheibenprothese nach einem der vorstehenden Ansprüche,
**dadurch gekennzeichnet, dass** der innere Hohlraum Drucksensoren umfasst.

4. Bandscheibenprothese nach einem der vorstehenden Ansprüche,
**dadurch gekennzeichnet, dass** die äußerste Schicht der äußeren wasserdichten verformbaren Hülle mit einem starren Material verstärkt ist, vorzugsweise ausgewählt aus Polyester und Polyethylen, einzeln oder in Kombination verwendet.

5. Bandscheibenprothese nach einem der vorstehenden Ansprüche, **dadurch gekennzeichnet, dass** die vertikalen Wände (2) mindestens eine vertikale Wand umfassen, die eine mehrfach gefaltete Form aufweist, die federähnliche Eigenschaften verleiht.

6. Bandscheibenprothese nach einem der vorstehenden Ansprüche, **dadurch gekennzeichnet, dass** die vertikalen Wände (2) mindestens eine vertikale Wand umfassen, die eine röhrenförmige Form aufweist.

7. Bandscheibenprothese nach einem der vorstehenden Ansprüche, **dadurch gekennzeichnet, dass** die Wasserdichte in dem inneren Hohlraum konstant ist.

8. Bandscheibenprothese nach einem der vorstehenden Ansprüche,
**dadurch gekennzeichnet, dass** die vertikalen Wände (2) Öffnungen (3) mit einer größeren Abmessung zwischen 1 mm und 6 mm umfassen.

9. Bandscheibenprothese nach einem der vorstehenden Ansprüche,
**dadurch gekennzeichnet, dass** die äußere wasserdichte verformbare Hülle aus einem 3D-druckbaren Material hergestellt ist.

10. Bandscheibenprothese nach einem der vorstehenden Ansprüche, **dadurch gekennzeichnet, dass** die vertikalen Wände (2) aus demselben Material hergestellt sind wie die periphere Trennmembran (1).

11. Bandscheibenprothese nach einem der vorstehenden Ansprüche,
**dadurch gekennzeichnet, dass** die ungiftige Salzlösung physiologisches Serum ist.

12. Bandscheibenprothese nach einem der vorstehenden Ansprüche, **dadurch gekennzeichnet, dass** sie Sensoren umfasst, die in der Lage sind, Parameter der Bandscheibenprothese zu messen und den Messwert an eine externe Vorrichtung drahtlos zu übertragen, wobei die Parameter vorzugsweise aus Druck, pH-Wert und Salzgehalt ausgewählt sind.

## Revendications

1. Prothèse de disque intervertébral comprenant une enveloppe externe multicouche déformable et étanche entourant une membrane de séparation périphérique (1) délimitant un espace extérieur et une cavité interne,
dans laquelle la cavité interne est séparée par des parois verticales (2) délimitant au moins quatre compartiments de cavité (5) conçus pour être remplis d'une solution saline non toxique, et
dans laquelle les parois verticales (2) comprennent des orifices (3) et/ou des membranes poreuses conçues pour induire l'écoulement de la solution saline depuis un compartiment de cavité (5) vers un autre lors d'une application hétérogène d'une pression comprise entre 0,05 et 3 MPa pendant une durée comprise entre 10 secondes et 60 minutes, et pour retourner dans son compartiment d'origine (5) lors de la suppression de ladite pression pendant une durée comprise entre 10 secondes et 180 minutes, et
dans laquelle les parois (2) sont réparties de manière asymétrique dans la cavité interne de sorte qu'une partie postérieure de la cavité interne comprend des compartiments de cavité (5) moins nombreux et/ou plus grands qu'une partie antérieure de la cavité interne ;
dans laquelle les parois (2) sont ancrées dans la couche la plus extérieure de l'enveloppe externe déformable et étanche.

2. Prothèse de disque intervertébral selon la revendication 1, **caractérisée en ce que** l'enveloppe externe déformable et étanche comprend quatre couches.

3. Prothèse de disque intervertébral selon l'une quelconque des revendications précédentes, **caractérisée en ce que** la cavité interne comprend des capteurs de pression.

4. Prothèse de disque intervertébral selon l'une quelconque des revendications précédentes, **caractérisée en ce que** la couche la plus externe de l'enveloppe externe déformable et étanche est renforcée par un matériau rigide, de préférence choisi parmi polyester et polyéthylène, utilisés séparément ou en combinaison.

5. Prothèse de disque intervertébral selon l'une quelconque des revendications précédentes, **caractérisée en ce que** les parois verticales (2) comprennent au moins une paroi verticale présentant une forme pliée de multiple fois conférant des propriétés de ressort.

6. Prothèse de disque intervertébral selon l'une quelconque des revendications précédentes, **caractérisée en ce que** les parois verticales (2) comprennent au moins une paroi verticale présentant une forme tubulaire.

7. Prothèse de disque intervertébral selon l'une quelconque des revendications précédentes, **caractérisée en ce que** la densité de l'eau dans la cavité interne est constante.

8. Prothèse de disque intervertébral selon l'une quelconque des revendications précédentes, **caractérisée en ce que** les parois verticales (2) comprennent des orifices (3) dont la plus grande dimension est comprise entre 1 mm et 6 mm.

9. Prothèse de disque intervertébral selon l'une des quelconques revendication précédentes, **caractérisée en ce que** l'enveloppe externe déformable et étanche est constituée d'un matériau imprimable en 3D.

10. Prothèse de disque intervertébral selon l'une quelconque des revendications précédentes, **caractérisée en ce que** les parois verticales (2) sont constituées du même matériau que la membrane de séparation périphérique (1).

11. Prothèse de disque intervertébral selon l'une quelconque des revendications précédentes, **caractérisée en ce que** la solution saline non toxique est du sérum physiologique.

12. Prothèse de disque intervertébral selon l'une des revendications précédentes, **caractérisée en ce qu'**elle comprend des capteurs capables de mesurer des paramètres de la prothèse de disque intervertébral et de communiquer sans fil la valeur mesurée à un dispositif externe, dans laquelle lesdits paramètres sont de préférence choisis parmi pression, pH et salinité.
